Europäisches Patentamt

European Patent Office

Office européen des brevets

(19)

(11) Veröffentlichungsnummer: **0 075 791**
**B1**

(12) # EUROPÄISCHE PATENTSCHRIFT

(45) Veröffentlichungstag der Patentschrift:
09.04.86

(21) Anmeldenummer: 82108555.2

(22) Anmeldetag: 16.09.82

(51) Int. Cl.⁴: **A 61 L 15/01,** C 08 L 1/24,
C 08 J 9/00, A 61 F 13/00

(54) Absorbierender Wundverband und Verfahren zu seiner Herstellung.

(30) Priorität: 30.09.81 DD 233684
11.11.81 DD 234773

(43) Veröffentlichungstag der Anmeldung:
06.04.83 Patentblatt 83/14

(45) Bekanntmachung des Hinweises auf die Patenterteilung:
09.04.86 Patentblatt 86/15

(84) Benannte Vertragsstaaten:
AT BE CH DE FR GB IT LI NL SE

(56) Entgegenhaltungen:
AT - B - 331 993
AT - B - 363 578
DE - A - 2 326 102
DE - A - 2 364 628
DE - A - 2 403 269
DE - A - 2 638 654
DE - A - 2 750 622
US - A - 4 169 121

Die Akte enthält technische Angaben, die nach dem
Eingang der Anmeldung eingereicht wurden und die
nicht in dieser Patentschrift enthalten sind.

(73) Patentinhaber: VEB Leipziger Arzneimittelwerk,
Elisabeth-Schumacher-Strasse 54-56, DDR-7050 Leipzig
(DD)

(72) Erfinder: Dautzenberg, Horst, Dr., Moldaustrasse 2b,
DDR-1530 Teltow (DD)
Erfinder: Loth, Fritz, Dr., Elbestrasse 36c,
DDR-1530 Teltow (DD)
Erfinder: Borrmeister, Bodo,
Ernst-Thälmann-Strasse 86,
DDR-1502 Potsdam-Babelsberg (DD)
Erfinder: Bertram, Dieter, Dr., Strasse des 18.
Oktober 8/75, DDR-7010 Leipzig (DD)
Erfinder: Lettau, Herbert, Dr., Block 036/03,
DDR-4090 Halle-Neustadt (DD)
Erfinder: Stamberg, Jiri, Dr., u. petrini 5, Praha 6 (CS)
Erfinder: Péska, Jan, Dr., Varsavska 13, Praha 2 (CS)

(74) Vertreter: Patentanwälte Beetz sen. - Beetz jun. Timpe -
Siegfried - Schmitt-Fumian, Steinsdorfstrasse 10,
D-8000 München 22 (DE)

## Beschreibung

Die Erfindung betrifft einen absorbierenden Wundverband, der zum Abdecken und zur Reinigung von stark nässenden, infektiösen Wunden sowie betroffener Flächen bei Haut- und Gewebeerkrankungen dient, sowie ein Verfahren zur seiner Herstellung.

Es ist bekannt, daß ein moderner Wundverban, der bei Defektwunden unterschiedlicher Genese, bedonders aber auch bei Problemindikationen wie Ulcus cruris, Dekubitalulzera, diabetischem Gangrän, chronisch infizierten Wunden und Verbrennungswunden 2. und 3.

Grades eingesetzt werden kann, toxikologisch unbedenklich, immunologische yerträglich, problemlos herstellbar, lagerungsfähig und sterilisierbar sein muß; des weiteren muß er zur Ventilation der Wunde eine ausreichende Gaspermeabilität aufweisen, ohne mit dem Wundschorf zu verkleben auf der Wundflache gut haften, durch ein hohes Aufnahmevermögen für wundheilungsstörende Faktoren (Wundexsudat, Bakterien, nekrotisches Zellmaterial) intensiv wundreinigend und dadurch infektionshemmend bzw. -verhindernd sein und darüber hinaus granulations- und/oder epithelisationsfördernd wirken. Es ist ebenso bekannt, daß die konventionellen Wundverbände, die meistens Gewebe oder Gewirke aus Natur- oder Synthesefasern, vor allem aus regenerierter Cellulose in Form von Kurz- oder Langfasern, darstellen, diese Forderungen nur sehr unvollkommen erfüllen; insbesondere verkleben sie mit der Wunde bzw. mit dem Wundschorf, so daß beim Verbandwechsel der schützende Wundschorf aufgerissen, die Wunde irritiert und der Heilungsprozeß unterbrochen und naturgemäß verlängert wird; des weiteren ist ihre Saugfähigkeit begrenzt, wobei insbesondere Bakterien nicht aufgenommen werden, was zur Folge hat, daß eine Infektionseindämmung oft nur durch den zusätzlichen Einsatz antibakterieller Wirkstoffe wie Antibiotika oder Sulfonamide möglich ist, Zur Überwindung dieder Nachteile wurden Wundverbände entwickelt (z. B. DD—PS 97547, DE—OS 1209702, DE—OS 1254295, DE—OS 1492409, DE—OS 1629425, US—PS 2923298, US—PS 3012918, US—PS 3043301, US—PS 3285245, US—PS 3434472, US—PS 3438371, US—PS 3441021, US—PS 3446208, US—PS 3457919, US—PS 3579628, US—PS 3750666), die wundseitig mit einer nichtadhärenten, flexiblen perforierten Kunststoffolie aus Polyethylen, Polypropylen, Polyvinylchlorid, Polyvinylacetat, Polyethylenterephthalate, Ethylen/Vinylacetat-Copolymeren, Polyacrylaten oder Polyvinylpyrrolidon unterleft sind.

Es sind ferner auch Wundverbände bekannt, die statt der perforierten Kunststoffolien wundseitig gleichartig wirkende dünne, perforierte Metallfolien aus Silber, Aluminiumn oder Zink tragen (z. B. DE—OS 1161384, DE—OS 1417379, US—PS 2934066). Derartige Verbände verkleben nicht mit der Wunde; sie lassen andererseits das Wundexsudat durch die Perforierung hindurch, das von den hinterlegten Saugschichten (Zellstoff, Preßwatte, Baumwollfilz) aufgenommen werden kann. Abgesehen davon, daß die Saugfähigkeit derartiger Wundverbände nicht grundsätzlich erhöht ist, können die schmierig-eitrigen Exsudate von stark infizierten Wunden auch die Öffnungen verstopfen, wodurch dann Exsudattaschen entstehen, die ausgezeichnete Wachstumsmöglichkeiten für Bakterien bieten; bei den metallunterlegten Verbandfolien kommt als weiterer Nachteil noch eine nicht gänzlich auszu schließende Scheuerwirkung hinzu.

Eine erhöhte Saugfähigkeit der Wundverbände wird durch den Einsatz solcher natürlicher oder synthetischer Materialien erreicht, die einerseits wasserunlöslich sind, zum anderen aber mit Wasser quellen.

Derartige Materialien sind z. B. Chitin und Chitosan (MIT Sea Grant Rep. MIT SG 1978, Proc. Int. Conf. Chitin/Chitosan 1977, S. 296—305), Collagen (DD—PS 56587, GB—PS 1195062, US—PS 3471598, US—PS 3491760, US—PS 3800792, US—PS 3939831, US—PS 4089333), naßvernetzte Cellulosefasern, besonders formalisierte Baumwoll- und Kunstseidefasern (DE—OS 1492365), die zweckmäßigerweise noch 5—20 Mass-% des Na-Salzes der Carboxymethylcellulose (Na-CMC) enthalten (DE—OS 2638654), Mischfasern aus Celluloseregenerat und Na-CMC (US—PS 3858585) bzw. vernetzte CMC-Produkte (DE—OS 2357079), Hydrolyseprodukte vernetzter Copolymerer aus Vinylestern und ungesättigten Carbonsäuren (DE—OS 2653135), vernetzte Polyacrylamide und sulfonierte Polystyrole (DE—OS 1617998, DE—OS 1642072, US—PS 3669193), vernetzte Poly-N-vinylpyrrolidone and -morpholinone (US—PS 3810468), vernetzte Dextrane, Stärken, Dextran- und Stärkederivate (DD—PS 109513, DE—OS 2403269) sowie Schaumstoffe aus Harnstoff/Formaldehyd- bzw. Melamin/Formaldehyd-Harsen (DE—OS 1246173, DE—OS 1247553), die zusätzlich Butadien/Styrol- oder Butadien/Acrylnitril-Copolymere enthalten können, (US—PS 3314425) und solche aus Polyurethanen (DE—OS 2103590, GB—PS 1065994), GB—PS 1253845, US—PS 3157178, US—PS 3648692, US—PS 3975567, US—PS 3978855). Die Hydrolyseprodukte der vernetzten Vinylacetate/ Methacrylat-Copolymerem können z. B. bis zum 100fachen, die vernetzten Polyacrylamide bis zum 70fachen und die vernetzten Poly-N-vinylpyrrolidone wenigstens das 15fache ihres Eigengewichtes Wasser bzw. Wundexsudat aufnehmen.

Die meisten Wundverbände sind Gewebe oder Gewirke faserförmiger Erzeugnisse, d. h. sie gelangen in Form vorgefertiger Flächengebilde zur Anwendung. Noch vergleichsweise selten erfolgt die Abdeckung von Wunden mit partikelförmigen Materialien wie Streupulvern oder dergleichen, obwohl gerade sie bei unebenen und zerklüfteten Wunden gegenüber den flächenförmigen Verbänden große Vorteile aufweisen.

Zur Wundabdeckung geeignete partikelförmige Materialien sind z. B. in der DE—OS 2403269 beschrieben. Dabei handelt es sich um Mikroperlen vernetzter Polysaccharide und Polysacchariddderivate, besonders vernetzter Dextrane, die unmittelbar auf die Wunde aufgetragen werden. Es ist weiterhin bekannt, daß für den gleichen Zweck auch sphärische Regeneratcelluloseteilchen eingesetzt werden können. Beide Materialien besitzen ein hohes Sorptionsvermögen für Wundsekrete und vermögen außerdem Bakterien, Pilze, Toxine, Entzündungsmediatoren (Prostaglandine) und Plasmaproteine (Fibrinogen-Spaltprodukte) von der Wundoberfläche zu entfernen.

Trotz vieler nachweislich positiver Eigenschaften besitzen diese beiden patikelförmigen Materialien noch einige Nachteile, die ihre verbreitete Anwendung einschränken. Die aus vernetzten Dextranen aufgebauten Teilchen, deren Gelmatrix weder im trockenen noch im feuchten Zustand echte Poren aufweist, quellen beim Kontakt mit Wasser bzw. wäßrigen Lösungen unter stärker Volumenvergrößerung und ergeben eine Gelschicht, innerhalb deren die Gaspermeabilität herasbgesetzt ist. Dieser Nachteil ist bei den Regeneratcelluloseteilchen, die eine makroporöse Struktur besitzen und demzufolge Wasser ohne jede Quellung aufnehmen, zwar überwunden, aber gerade ihre Porosität bewirkt, daß auch nichtwäßrige Medien, z. B. organische Solventien, aufgesaugt werden, was sich bei bestimmten Anwendungsformen ungünstig auswirken kann; ihre Sterilisation mit γ-Strahlen ist außerdem mit einer Gelb- bis Braunfärbung verbunden.

Die vorstehend genannten partikelförmigen Materialien können nach an sich bekannten Verfahren hergestellt werden, die Cellulosepartikel z. B. durch Ansäuren emulgierter Celluloselösungen (SE—PS 382066, US—PS 3597350), durch Eintropfen von Viskoselösungen in geeignete Koagulationsbäder (JP—PS 73.21738, JP—PS 73.4082, JP—PS 73.60753), durch kurzzeitiges Erhitzen von partikelförmigen Celluloseestern, wie z. B. Celluloseacetate, in Siliconöl auf 290—300°C (JP—PS 78.07759, JP—PS 78.86749) oder durch thermische Koagulation von Viskosedispersionen (CS—US 172640, DD—PS 118887, US—PS 4055510).

Die nach diesen Verfahren zugänglichen Cellulosepartikel ergeben allerdings nur dann einen saugfähigen Wundverband obiger Qualität, wenn sie so getrocknet werden, daß ihre Makroporosität erhalten bleibt. Nach dem bisherigen Stand der Technik gelingt dies nuir mit speziellen Trocknungsverfahren, die aufwendig und unwirtschaftlich sind. Der Erfindung liegt entsprechend die Aufgabe zugrunde, einen Wundverband auf der Basis eines partikelförmigen Cellulosematerials und einfache Verfahren zur seiner Herstellung anzugeben, der atoxische und gewebeverträglich ist, der Wundform bzw. den betroffenen Haut- und Gewebepartien, auch unebenen und zerklüfteten Wunden, gut angepaßt werden kann, aus stark nässenden Wunden das Exsudat schnell und für den Patienten schonend entfernt, neben dem Exsudat auch Bakterien, Fungi, Toxine, Proteine und Prostaglandine bindet, der sterilisierbar und nachsterilisierbar ist und die Granulation und Epithelisation fördert, aber außerdem infolge einer echten bzw. latenten Makroporosität nur in geringem Maße zur Verquellung neigt, so daß die Gaspermeabilität der wundbedeckenden Schicht weitgehend unbeeinträchtigt bleibt, und der schließlich· unfolge einer günstigen Rohstoffbasis und einer einfachen Herstellbarkeit wirtschaftlich vorteilhaft ist und in breitemn Umfang zum Einsatz gelangen kann.

Die Aufgabe wird erfindungsgemäß durch einen absorbierenden Wundverband aus einem partikelförmigen Cellulosematerial gelöst, das dadurch gekennzeichnet ist, daß die Partikel aus einem Gemisch von Regeneratcellulose und 3—30 Masse-% eines Carboxylatgruppen enthaltenden Polysaccharidderivats und gegebenenfalls 5—30 Masse-% Glycerin und/oder Polyalkylenglycol bestehen und nach der Benutzung mit wässerigen Flüssigkeiten eine makroporöse Struktur aufweisen. Vorteilhafte Ausführungsformen sind gegenstand der Unteransprüche. Der Erfindung liegt die überraschende Feststellung zugrunde, daß trockene partikelförmige Produkte aus gemischen von Regeneratcellulose und einem Carboxylatgruppen enthaltenden Polysaccharidderivat, nachfolgend kurz Polysaccharidderivat genannt, einen absorbierenden Wundverband der angestrebten Qualität ergeben, überraschenderweise auch deshalb, weil die der Regeneratcellulose zugemischten Polysaccharidderivate an sich plasmakoagulierend und damit hämostatisch wirken, was eine unerwünschte Verkrustung der Wunde zur Folge haben sollte.

Eine derartige Verkrustung tritt aber nicht ein; die auf de Wunde gebrachten erfindungsgemäse Wundverband, der aus sphärischen Teilchen oder einem Granulat mit einem Partikeldurchmesser >10 μm besteht, saugt wäßrige Wundsekrete beliebiger Konsistenz und mit ihnen zugleich Bakterien, Pilze, Toxine, Proteine und Entzündungsmediatoren rasch und weitgehend schmerzfrei auf, so daß Wundinfektionen eingedämmt bzw. verhindert werden und die Wunde rasch abtrocknet und dabei aber elastisch bleibt. Die körnige Struktur der Partikel, die auch im exsudatgesättigten Zustand erhalten bleibt, regt außerdem die Granulation und Epithelisation an, und die hohe Formstabilität, die durch die vergleilchsweise geringe Quellung der Partikel erreicht wird, garantiert eine gute Ventilation der Wunde.

Die Polysaccharidderivate, die mit Regeneratcellulose vermischt und verformt den erfindungsgemäßen Wundverband ergeben, müssen zu wenigstens 50% in Wasser löslich sein; geeignet sind z. B. Natriumcarboxymethylcellulose, Natriumcarboxymethylstärke und Natriumalginat. Ihr Anteil im Polymergemische beträgt 3 bis 30%.

Die erfindungsgemäßen wunverbände aus gemischen von Regeneratcellulose und Polysaccharidderivaten zeichnen sich gegenüber den sphärischen Partikeln aus reiner Regeneratcellulose dadurch aus, daß die nach der Herstellung erforderliche Trocknung in konventioneller Weise durchgeführt werden kann.

Zwar kollabiert dabei das ursprünglich vorhandene Porensystem unter Schrumpfung der Partikel, wird aber bei der Benetzung mit Wasser unter Anwendungsbedingungen weitgehend regeneriert, so daß das Sorptionsvermögen der Partikel nicht nur dem reiner Cellulosepartikel gleicht sondern es sogar übdertrifft.

Dabei machen sich der hydrophile Charakter der eingesetzten Polysaccharidderivate und ihr chromatographisches Trennvermögen zusätzlich positiv bemerkbar.

Während die reinen Cellulosepartikel Keime im wesentlichen nur in die interpartikulären Kapillarräume aufnehmen können, in denen sie nur relativ locker festgehalten werden, bewirken die besondere innere Struktur der erfindungsgemäßen Partikel und der ionische Charakter der Polysaccharidderivate eine erheblich festere Bindung. Ein weiterer Vorteil besteht darin, daß das Wasseraufnahmevermögen und die Kinetik der Wasser-bzw. Exsudataufnahme in relativ weiten Grenzen über den Herstellungsprozeß eingestellt werden können und somit eine bessere Anpassung an verschiedenartige Anforderungen möglich ist.

Ein erheblicher Vorteil ist weiterhin, daß die erfindungsgemäßen partikelförmigen Produkte ihre positive Wirkung auch in Gegenwart von Glycerin oder Polyalkylenglykolen behalten, wodurch die Herstellung pastenförmiger Zubereitungen möglich ist. Daneben hat sich gezeigt, daß sich durch Aufbringen von Glycerin oder Polyethylenglykol in einer Menge von 5 bis 30% eine Schädigung bei der strahlenchemischen Sterilisation verhindern läßt, die anderenfalls unter Verschlechterung der Produkt parameter und Gelbfärbung eintritt.

Der erfindungsgemäße Wundverband wird gewöhnlich in einer Schichtdicke bis zu 5 mm auf die Wunde gebracht und gegebenenfalls durch einen leichten konventionellen Druckverband gesichert. Der Verbandwechsel, der nach der Sättigung der Partikel mit Wundexsudat, spätestens aber nach 14 bis 20 Stunden erfolgen sollte, ist z. B. durch Abspülen der Partikelschicht mit Wasser oder physiologischer Kochsalzlösung und erneutes Auftragen des partikelförmigen Materials denkbar einfach und mühelos.

Zu einem Wundverband der vorstehend beschriebenen Qualität gelangt man erfindungsgemäß z. B. dadurch, daß man einer technisch überlichen Viskoselösung ein Polysaccharidderivat obiger Spezifikation in einem Antiel von 5 bis 50%, bezogen auf die Gesamtmasse an Polymermischung in der Viskoselösung, zumischt, diese Mischung thermisch koaguliert, das Koagulat reinigt, ggfs. mit 5 bis 30% eines Polyalkylenglykols oder Glycerin benetzt und trocknet.

Die partikelstruktur der Produkte kann vor oder nach der Koagulation erzeugt werden. Nach einer speziellen Ausfunrungsform des erfundungsgemäßen verfahrens erfolgt die Partikelbildung, indem eine das zusätzlich Polysaccharidderivat enthaltende Viskoselösung in geeigneter Weise nach an sich bekannten Techniken, z. B. durch Dispergieren in einer mit Wasser nicht mischbaren organischen Flüssigkeit, tröpfchenförmig verteilt und durch Erwärmung auf Temperaturen von 50 bis 100°C innerhalb von 30 bis 90 min thermisch koaguliert und regeneriert wird.

Alternativ wird nach einer weiteren Verfahrensvariante die das Polysaccharidderivat enthaltende Viskoselösung unter vergleichbaren Bedingungen, aber ohne vorherige feine Zerteilung, behandelt, wobei das Koagulat in Form von Platten, Stäben oder Blöcken anfällt, die dann in geeigneter Weise mechanisch zerkleinert werden.

Die erhaltenen feinteligen, krümeligen Massen ergeben nach Auswaschen und Trocknung pulverigkörnige Produkte, die zu Erzielung des gewünschten Korngrößenverteilung ggf. noch einer Trockenmahlung zu unterwerfen sind.

Die erhaltenen partikelförmigen Produkte werden zur Reinigung und gleichzeitung zur Steuerung der Produkteigenschaften einer Reihe von Waschoperationen mit niedermolekularen Alkoholen, vorzugsweise Methanol oder Ethanol, oder Ketonen, vorzugsweise Aceton, mit wäßrigen Lösungen dieser Alkohole und/oder Ketone und/oder mit Wasser oder wäßrigen Lösungen, wie z. B. werdünnten Säuren oder Alkalien, unterworfen, wobei so verfahren wird, daß 5 bis 50% des zugemischten Polysaccharidderivats ausgewaschen werden.

Die Trocknung dieser Produkte kann nach bekannten einfachen Verfahren erfolgen,m z. B. auf einem Wirbelbett-Trockner, wozu man die Partikel vorher durch Zentrifugieren ode Absaugen von der überschüssigen letzten Waschflüssigkeit befreit, die zweckmäßigerweise ein nichtwäßriges oder zumindesdt zum Teil nichtwäßriges Medium sein sollte und ggf. bereits eine Zusatz von Polyalkylenglykol oder Glycerin enthält.

Als zugemischte Polysaccharidderivate kommen Natriumcarboxymethylcellulose, Natriumcarboxymethylsträrke und Natriummalginat in Betract.

Nach einer bevorzugten Ausführungsform des Verfahrens wird als Polysaccharidderivat Natriumcarboxymethylcellulose mit einem mittleren Substitutionsgrad (DS) von 0,4 bis 1,0 eingesetzt.

In Abhängigkeit von Art und Menge der eingebrachten natriumcarboxymethylcellulose sowie von den Verfahrensbedingungen während der Nachbehandlung und Trocknung erhält man mehr oder minder stark geschrumpfte, weitgehend isometrische Partikel, die zwischen 1,5 und 20 ml Wasser pro Gramm Trockensubstanz aufzunehmen vermögen, wobei die durch Dispergierung gewonnen Teilchen annähernd spärisch sind und eine vergleichsweise glatte Obefläche aufweisen und die anders hergestellten Teilchen eine unregelmäßigere Gestalt sowie eine rauhere Oberfläche zeigen, ohne daß dadurch aber die wesentlichen Produktkenngrößen nachteilig beeinflußt werden.

Grundsätzlich kann man die aus den Polymergemischen besehenden erfindungsgemäßen Partikel in der gleichen Weise schonend trocknen wie die reinen Regeneratcellulosematerialien. In diesem Falle erhält man Produkte, die weniger geschrumpft sind und bereits im trackenen Zustand eine hohe, Makroprosität aufweisen. Setzt man die Polysaccharidderivate in gelöster Form und in einer solchen Konzentration der Viskoselösung zu, daß dadurch eine Herabsetzung der Gesamtpolymerkonzentration der Lösung resultiert, oder entfernt man durch die nachfolgenden Behandlungsschritte größere Mengen des Polysaccharidderivats aus den feuchten Partikeln, dann resultieren nach einer schonenden Trocknung Materialien, die gegenüber den reinen Regeneratcelluloseprodukten ein stark erhöhtes Hohlraumvolumen besitzen.

Die folenden Beispiele beschreiben Herstellung und Anwendung des erfindungsgemäßen Wundverbands.

## Beispiel 1

Zur Herstellung des erfindungsgemäßen Wundverbandes wurde ein gemisch aus 50 g Viskose (8% Cellulose, 6% Natriumhydroxid, Xanthogenierung mit 35% Schwefelkohlenstoff, bezogen auf Cellulose) und 50 g wäßriger 4%iger Lösung einer Natrium-Carboxymethylcellulose (Na-CMC) mit einem metttleren Substitutionsgrad (DS) von 0,3 in 150 ml Chlorbenzol das 0,05% Ölsäure als Emulgator enthielt, unter Rühren dispergiert. Die erhaltenen Tröpfchen wurden unter weiterem Rühren 30 min bei 90°C koaguliert. Das dabei entstandene Produkt aus hochgequollenen sphärischen Teilchen wurde durch Absaugen vom Chlorbenzol und Wasser abgetrennt. Zur Aufarbeitung wurden — wie auch in einigen nachfolgenden Beispielen — jeweils Protionen zu 20 g des erhaltenen feuchten Rohprodukts mit einem Polymergehalt von etwa 1,3 g eingesetzt. Es wurde nach folgenden Verfahrensvarianten gereinigt:

a) Die Teilchen wurden viermal mit je 100 ml wäßrigem Methanol (80 Vol.-%) alkali- und salzfrie gewaschen, abschließend 10 min mit 70 ml Ethanol behandelt.

b) Die Teilchen wurden folgenden Verfahrensschritten unterworfen

| | | |
|---|---|---|
| 100 ml Wasser, | 10 min, | 90°C; |
| 100 ml Wasser, | 10 min, | 90°C; |
| 100 ml Wasser, | 10 min, | Raumtemperatur; |
| 70 ml Ethanol, | 10 min, | Raumtemperatur. |

c) Die Teilchen wurden nach Verfahrensvariante b) gereinigt, jedoch mit dem Unterschied, daß ein drittes Mal mit Wasser von 90°C eluiert wurde.

d) Die Teilchen wurden folgenden Verfahrensschritten unterworfen:

| | | |
|---|---|---|
| 100 ml wäßriges Methanol, 80 Vol.-%, | 10 min, | Raumtemperatur; |
| 100 ml wäßriges Methanol, 80 Vol.-%, | 10 min, | Raumtemperatur, |
| 100 ml Wasser, | 10 min, | 90°C; |
| 100 ml Wasser, | 10 min, | Raumtemperatur; |
| 70 ml Ethanol, | 10 min, | Raumtemperatur. |

Die gereinigten sphärischen Teilchen wurden abgesaugt und bei 105°C getrocknet und ergaben dann die nachstehenden Analysendaten:

| Verfahrensvariante | GMC-Gehalt (%) | Wasseraufnahme (ml/g) | Löslicher Antiel (%) |
|---|---|---|---|
| a) | 33,3 | 5,9 | 7,4 |
| b) | 26,4 | 6,2 | 3,2 |
| c) | 15,9 | 7,3 | 1,5 |
| d) | 28,1 | 7,5 | 1,0 |

Die Wasseraufnahme wurde, auch in den folgenden Beispielen, nach dem Zentrifugierverfahren bestimmt (Cell. Chem. Technol. 21 (1978) 419—428) und entspricht der im Porensystem der Cellulosepartikel zurückgehaltenen Wassermenge (Wasserretention).

## Beispiel 2

Zur Herstellung eines erfindungsgemäßen Wundverbands wurden sphärische Teilchen analog Beispiel 1, jedoch unter Einsatz einmer Na-CMC mit einem mittleren substitutionsgrad von 0,5 als Mischungskomponente, in mehreren getrennten Chargen hergestellt. Die sphärishen Teilchen dieser Chargen wurden nach der Verfahrensvariante b) von Beispiel 1 gereinigt und ergaben nach Trocknung bei einer Restlöslichkeit von 2 bis 4% folgende innerhalb der Fehlergrenzen korrelierende Werte für die Wasseraufnahme:

# 0 075 791

|  | Wasseraufnahme ml/g |
|---|---|
| Charge 1 | 6,1 |
| Charge 2 | 6,1 |
| Charge 3 | 5,9 |
| Charge 4 | 6,5 |
| Charge 5 | 6,9 |
| Charge 6 | 6,6 |
| Charge 7 | 6,3 |

## Beispiel 3

Zur Herstellung eines erfindungsgemäßen Wundberbands wurden sphärische Teilchen analog Beispiel 1, jedoch unter Einsatz einer Na-CMC mit einem mittleren Substitutionsgrad von 0,7 als Mischungskomponente, hergestellt und nach zwei verschiedenen Verfahrensvarianten nachbehandelt und gereinigt:

a) Die Teilchen wurden folgenden Verfahrensschritten unterworfen:

| 100 ml Methanol, | 10 min, | Raumtemperatur; |
|---|---|---|
| 100 ml Methanol, | 10 min, | Raumtemperatur; |
| 100 ml Wasser, | 10 min, | 90°C; |
| 100 ml Wasser, | 10 min, | Raumtemperatur; |
| 70 ml Ethanol, | 10 min, | Raumtemperatur. |

b) Die Teilchen wurden folgenden Verfahrensschritten unterworfen:

| 100 ml Wasser, | 10 min, | 90°C; |
|---|---|---|
| 100 ml Wasser, | 10 min, | 90°C; |
| 100 ml Wasser, | 10 min, | Raumtemperatur; |
| 70 ml Ethanol, | 10 min, | Raumtemperatur. |

Die gereinigten und nachbehandelten Teilchen wurden von der flüssigen Phase getrennt und bei 105°C getrocknet und ergafen dann folgende Werte für die Wasseraufnahme:

| Verfahrensvariante | Wasseraufnahme (ml/g) | Löslicher Anteil (%) |
|---|---|---|
| a) | 9,6 | 3,4 |
| b) | 5,2 | 2,0 |

## Beispeil 4

Zur Herstellung eines erfindungsgemäßen Wunderbands wurden 25 g einer gemischs aus 12,5 g Viskose und 12,5 g wäßriger 4%iger Na-CMC (mittlere Substitutionsgrad 0,5) in ein zylindrisches Gefäß von etwa 10 mm Innendurchmesserr gegossen und 90 min bei 90°C thermische koaaguliert. Das Material wurde dann zu einem feinteilgen Granulat einer Teilchengröße von etwas 1 mm zerkleinert und nach Veriante a) von Beispiel 3 gereinigt. Das nach Trocknung bei 105°C erhaltene pulverig-körnige Produkt zeigte eine Wasseraufnahme von 7,6 ml/g.

## Beispeil 5

Zur Herstellung eines erfindungsgemäßen Wundverbands wurden sphärische Teilchen analog Beispiel 1, jedoch unter Einsatz einer mit Epichlorhydrin teilvernetzten Na-CMC (mittlerer Substitutionsgrad 0,7) mit einer Löslichkeit in Wasser von noch etwa 50% hergestellt. Die Mischungskomponente wurde dabei der Viskose, in 1n-Natronlauge weitgehend gelöst, zugemischt. Die erhaltenen Teilchen wurden nach

6

der Verfahrensvariante d) von Beispiel 1 gereinigt und zeigten nach Trocknung bei 105°C eine Wasseraufnahme von 14,0 ml/g bei 0,6% Restlöslichkeit.

## Beispiel 6

Entsprechend Beispiel 1 wurden sphärische Teilchen unter Zusdatz von Natriumcarboxymethylstärke und Natriumalginat hergestellt, nach Verfahrensvariante d) von Beispiel 1 gereinigt und bei 105°C getrocknet. Die Produkte wiesen folgende Eigenschaften auf:

| Polysaccharidderivat | Wasseraufnahme (ml/g) | Löslicher Auteil (%) |
|---|---|---|
| Natriumcarboxymethylstärke | 5,7 | 4,2 |
| Natriumalginat | 3,1 | 1,5 |

## Beispiel 7

Die stark sezernierende Wunde eines Patienten mit einem Ulcus cruris wurde mit einer ca. 3 mm starken Schicht eines erfindungsgemäßen Wundverbands bedeckt der aus trockenen, durch Bestrahlung mit γ-Strahlen sterilisierten sphärischen Regeneratcelluloseteilchen, die 10% Natriumcarboxymethylcellulose enthielten betand und eine mittlere Teilchengröße von 0,2 mm und eine Wasseraufnahme von 3,2 ml/g aufwies. Der Verband wurde täglich gewechselt, indem das vollgesaugt Material mit physiologischer Kochsalzlösung abgespült und eine neue, ca. 3 mm starke Verbandschicht aufgelegt wurde. Nach 5 Tagen war die Wunde deutlich gereingt und nahezu trocken, und nach 9 Tagen konnte ein konventioneller Verband anglegt werden.

## Beispiel 8

Eine infizierte oberflächliche offene wunde eines Patienten nach posttraumatischer Ostitis wurde mit einer ca. 3 mm starken Schicht eines Wundverbands bedeckt, der aus einem trockenen, durch Bestrahlung mit γ-Strahlen streilizierten granulat von Regeneratcelluloseteilen bestand, die 20% Natriumcarboxymethylcellulose und 5% Glycerin enthielten und eine Teilchengröße von 0,1 bis 0,5 mm und eine Wasseraufnahme von 4,6 ml/g aufwiesen.

Der Verband wurde täglich gewechselt, wobei der Wundverband mit einem Spatel abgetragen wurde. Nach 5 Tagen zeigten sich ein deutliches Abklingen der Entzündungserscheinungen und eine augenfällige Reinigung und gesunde Granulation der Wunde. Nach 8 Tagen konnte ein konventioneller Verband angelegt werden.

## Beispiel 9

Eine infizierte, stark nässende Wunde eines Patienten mit großflächiger Verbrennung wurde mit einer ca. 3 mm dicken Schicht eines Wundverbands bedeckt, der aus trockenen, durch Bestrahlung mit γ-Strahlen sterilisierten sphärischen Regeneratcelluloseteilchen bestand, die 10% Natriumcarboxymethylcellulose und 20% Polyethylenglykol (rel. Molekülmasse 600) enthielten und eine Teilchengröße von 0,2 bis 0,3 mm und eine Wasseraufnahme von 2,5 ml/g aufwiesen.

Der Verband wurde täglich gewechselt, wobei die Entfernung des vollgesaugten Materials ohne Verletzung des Wundgrundes gelang. Nach 7 Tagen war die Wunde sauber und trocken, und es begann vom Rande her die Epithelisierung des Defektes. Nach 12 Tagen war die Wunde transplantationsreif.

**Patentansprüche**

1. Absorbierender Wundverband aus einem partikelförmigen Cellulosematerial, dadurch gekennzeichnet, daß die Partikel aus einem Gemisch von Regeneratcellulose and 3 bis 30 Masse % eines Carboxylatgruppen enthaltenden Polysaccharidderivats und ggfs. 5 bis 30 Masse-% Glycerin und/oder Polyalkylenglykol bestehen und nach der Benetzung mit wässerigen Flüssigkeiten eine makroporöse Struktur aufweisen.

2. Wundverband nach Anspruch 1, dadurch gekennzeichnet, daß die Partikel sphärisch sind und einen Durchmesser >10 µm aufweisen.

3. Wundverband nach Anspruch 1 oder 2, dadurch gekennzeichnet, daß er ein Granulat darstellt.

4. Verfahren zur Herstellung der Wundverbände nach einem der Ansprüche 1 bis 3, gekennzeichnet durch

(A) Zugabe eines zu mehr als 50% wasserlöslichen, Carboxylatgruppen enthaltenden Polysaccharidderivats in fester Form oder in Form einer wässerigen Lösung zu einer Viskose technisch überlicher Zusammensetzung in einer Menge von 5 bis 50 Masse-%, bezogen auf die Gesamtmasse des Polymergemischs in der Viskoselöslung,

(B) feines Zerteilen des Gemisches aus Schritt (A),

(C) Koagulieren des Systems aus Schritt (B) bei erhöhter Temperatur,

(D) Waschen und Nachbehandeln der erhaltenen Partikel, ggfs, unter Auswaschen eines Teils des eingesetzten Polysaccharidderivats und

(E) Trocknen.

5. Verfahren zur Herstellung der Wundverbände nach einem der Ansprüche 1 bis 3, gekennzeichnet durch

(A) Zugabe eines zu mehr als 50% wasserlöslichen, Carboxylatgruppen enthaltenden Polysaccharidderivats in fester Form oder in Form einer wässerigen Lösung zu einer Viskose technisch überlicher Zusammensetzung in einer Menge von 5 bis 50 Masse-%, bezogen auf die Gesammtmasse des Polymergemischs in der Viskoselösung,

(B) Koagulieren des Gemisches aus Schritt (A) bei erhöhter Temperatur,

(C) Zerkleinern der erhaltenen Masse aus Schritt (B) auf die gewünschte Partikelgröße,

(D) Waschen und Nachbehandeln der erhaltenen Partikel, ggfs. unter Auswaschen eines Teils des eingesetzten Polysaccharidderivats und

(E) Trocknen.

6. Verfahren nach Anspruch 4 oder 5, dadurch gekennzeichnet, daß das Koagulat vor dem Trocknen (E) mit einem Polyalkylenglykol oder Glycerin in einer solchen Menge versetzt wird, daß deren Gehalt im Endprodukt, bezogen auf das Trockengewicht der Polymermasse, 5 bis 30 Masse-% beträgt.

7. Verfahren nach Anspruch 4 oder 6, dadurch gekennzeichnet, daß die feine Zerteilung in Schritt (B) durch Dispergieren des Gemischs aus Schritt (A) in einer nicht mit Wasser mischbaren organischen Flüssigkeit zu Dispersionspartikeln mit einem Durchmesser >10 µm vorgenommen wird.

8. Verfahren nach einem der Ansprüche 4 bis 7, dadurch gekennzeichnet, daß in Schritt (A) Natrium-Carboxymethylcellulose mit einem mittleren Substitutionsgrad von 0,4 bis 1,0 eingesetzt wird.

9. Verfahren nach einem der Ansprüche 4 bis 8, gekennzeichnet durch Verwendung von niedermolekularen aliphatischen Alkoholen oder Ketonen oder deren Gemischen mit Wasser und/oder wässerigen Lösungen in Schritt (D) und Auswaschen eines Teils des eingesetzten Polysaccharidderivats.

## Revendications

1. Pansement vulnéraire absorbant en matière cellulosique sous forme de particules, caractérisé en ce que les particules sont formées d'un mélange de cellulose régénérée et de 3 à 30% en masse d'un dérivé de polysaccharide contenant des groupes carboxylate et éventuellement de 5 à 30% en masse de glycérol et/ou de polyalkylèneglycol et qu'après le mouillage par des liquides aqueux, elles présentent une structure macroporeuse.

2. Pansement vulnéraire selon la revendication 1, caractérisé en ce que les particules sont sphériques et présentent un diamètre supérieur à 10 µm.

3. Pansement vulnéraire selon l'une des revendications 1 et 2, caractérisé en ce qu'il constitue un granulé.

4. Procédé pour la fabrication des pansements vulnéraires selon l'une des revendications 1 à 3, caractérisé par

(A) l'addition d'un dérivé de polysaccharide hydrosoluble à plus de 50%, contenant des groupes carboxylate, sous forme solide ou sous la forme d'une solution aqueuse, à une viscose de composition techniquement usuelle, à raison de 5 à 50% en masse par rapport à la masse totale du mélange de polymères dans la solution de viscose,

(B) la fine fragmentation du mélange provenant de l'étape (A)

(C) la coagulation, à température plus élevée, du système provenant de l'étape (B),

(D) le lavage et le posttraitement de la particule obtenue, éventuellement avec extraction par lavage d'une partie du dérivé de polysaccharide utilisé et

(E) le séchage.

5. Propcédé pour la fabrication des pansements selon l'une des revendications 1 à 3, caractérisé par (A) l'addition d'un dérivé de polysaccharide hydrosoluble à plus de 50%, contenant des groupes carboxylate, sous forme so lide ou sous la forme d'une solution aqueuse, à une viscose de composition techniquement usuelle, à raison de 5 à 50% en masse par rapport à la masse totale du mélange de polymères dans la solution de viscose.

(B) la coagulation, à température plus élevée, du mélange provenant de l'étape (A),

(C) la fragmentation de la masse obtenue de l'étape (B) à la grosseur de particules désirée,

(D) le lavage et le posttraitement de la particule obtenue, éventuellement avec extraction par lavage d'une partie du dérivé de polysaccharide utilisé et

(E) le séchage.

6. Procédé selon la revendication 4 ou 5, caractérisé en ce qu'avant le séchage (E), on ajoute au coagulat un polyalkylèneglycol ou du glycérol en quantité tell que la teneur dans le produit final, par rapport au poids sec de la masse de polymère, soit de 5 à 30% en masse.

7. Procédé selon la revendication 4 ou 6, caractérisé en ce que l'on effectue la fine fragmentation à l'étape (B) en dispersant le mélange provenant de l'étape (A) dans un liquide organique non miscible à l'eau en formant des particules de dispersion d'un diamètre supérieur à 10 µm.

8. Procédé selon l'une des revendications 4 à 7, caractérisé en ce qu'à l'étape (A), on utilise de la sodiumcarboxyméthylcellulose ayant un degré de substitution de 0,4 à 1,0.

9. Propcédé selon l'une des revendications 4 à 8, caractérisé par l'utilisation d'alcools ou de cétones aliphatiques à bas poids moléculaire ou de leurs mélanges avec de l'eau et/ou des solutions aqueuses, à l'étape (D) et par l'extraction par lavage d'une partie du dérivé de polysaccharide utilisé.

**Claims**

1. Absorbent wound dressing consisting of particular cellulosic material, characterized in that the particles consist of a mixture of regenerated cellulose and from 3 to 30 mass percent of a polysaccharide derivative containing carboxylate groups and optionally from 5 to 30 mass percent of glycerol and/or polyalkylene glycol and, after having been wetted with aqueous liquids, having a macroporous structure.

2. Wound dressing according to claim 1, characterized in that the particles are spherical and have a diameter larger than 10 µm.

3. Wound dressing according to claim 1 or 2, characterized in that it is in granular form.

4. A process for producing wound dressings according to one of claims 1 to 3, characterized by

(A) adding a more than 50% water-soluble, carboxylate groups-containing polysaccharide derivative in solid form or in the form of an aqueous solution to a viscose of technically customary composition in an amount of 5 to 50 mass percent, based on the total mass of polymer mixture in the viscose solution,

(B) finely dividing the mixture from step (A),

(C) coagulating the system from step (B) at elevated temperature,

(D) washing and after-treating the resulting particles, while optionally washing out a portion of the polysaccharide derivative employed, and

(E) drying.

5. Process for producing the wound dressings according to one of claims 1 to 3, characterized by

(A) adding a more than 50% water-soluble, carboxylate groups-containing polysaccharide derivative in solid form or in the form of an aqueous solution to a viscose of technically customary composition in an amount of 5 to 50 mass percent, based on the total mass of polymer mixture in the viscose solution,

(B) coagulating the mixture from step (A) at elevated temperature,

(C) comminuting the resulting composition from step (B) to the desired particle size,

(D) washing and after-treating the resulting particles, optionally while washing out a portion of the polysaccharide derivative employed, and

(E) drying.

6. Process according to claim 4 or 5, characterized in that prior to drying (E) the coagulate is mixed with a polyalkylene glycol or glycerol in an amount such that the content thereof in the final product is from 5 to 30 mass percent, based on the dry weight of the polymer composition.

7. Process according to claim 4 or 6, characterized in that the fine division in step (B) is effected by dispersing the mixture from step (A) in a water-immiscible organic liquid to form dispersion particles having a diameter larger than 10 µm.

8. Process according to one of claims 4 to 7, characterized in that in step (A) sodium carboxymethyl cellulose having an average degree of substituion from 0.4 to 1.0 is employed.

9. Process according to one of claims 4 to 8, characterized by using low molecular weight aliphatic alcohols or ketones, or mixtures thereof with water and/or aqueous solutions in step (D), and washing out a portion of the polysaccharide derivative employed.